# EUROPÄISCHE PATENTANMELDUNG

(11) **EP 2 476 393 A1**
(43) Veröffentlichungstag der Anmeldung: **18.07.2012**
(21) Anmeldenummer: 11150688.7
(22) Anmeldetag: 12.01.2011
(51) Int. Cl.: A61F 2/08

(54) **Bandschraube**

(71) Anmelder: Mondeal Medical Systems GmbH, 78570 Mühlheim a. d. Donau (DE)
(72) Erfinder: Deiler, Stephan, 85591, Vaterstetten (DE)
(74) Vertreter: Lermer, Christoph

(57) **Zusammenfassung**

Die Komponenten 1, 2 und 3 eines Implantats (Bandschraube) sind eine erste bzw. zweite Implantatkomponente 1 und 2, die jeweils einen Verbindungsabschnitt 11 bzw. 21 zur Verbindung mit der dritten Implantatkomponente 3 sowie ein Schraubengewinde 12 bzw. 22, insbesondere ein Spongiosa-Schraubengewinde, aufweisen. Die Verbindungsabschnitte 11 und 21 weisen jeweils einen ring- bzw. zylindermantelförmigen Eingriffabschnitt 110 bzw. 210 und einen ringförmigen Vorsprung 111 bzw. 211 auf. Die dritte Implantatkomponente 3 umfasst Halteringe 31 und 32, die mit den Verbindungsabschnitten 11 bzw. 21 verrastet werden. Das Implantat soll Verwendung finden bei frischen und alten Rupturen des SL-Bandes.

## Beschreibung

### TECHNISCHES GEBIET

Die vorliegende Erfindung betrifft ein Implantat zur Herstellung einer Bandverbindung zwischen einem ersten Knochen und einem zweiten Knochen umfassend: eine erste Implantatkomponente zur Befestigung am ersten Knochen; eine zweite Implantatkomponente zur Befestigung am zweiten Knochen; und ein Verbindungselement mit einem ersten Anschlussabschnitt und einem zweiten Anschlussabschnitt.

### STAND DER TECHNIK

Der Aufbau des Handgelenks des Menschen ermöglicht hohe Beweglichkeit. Die Verbindung der Knochen des Handgelenks miteinander zu einem stabilen Gefüge wird durch die Bänder des Handgelenkes hergestellt.

Kommt es zur Verletzung des komplizierten Bandapparats, resultiert daraus eine Gefügestörung. Bandverletzungen am Handgelenk, beispielsweise verursacht durch Unfälle mit Sturz, können zu Fehlstellungen der Handwurzelknochen und unbehandelt zu einer verstärkten Belastung der Gelenkflächen führen sowie deren Verschleiß fördern.

Besonders häufig betroffen ist die Bandverbindung zwischen dem Kahnbein und dem Mondbein (SL-Band; scapho-lunäres Band; dies ist die Bandverbindung zwischen Os scaphoideum und Os lunatum). Nach einer Ruptur des Bandes weichen die beiden Knochen auseinander. Der Gelenkspalt zwischen den beiden Knochen vergrößert sich. Eine spontane Heilung des Bandes ist wegen der zu großen Distanz und der vergrößerten Bewegungsumfänge der beiden Knochen nicht mehr möglich. Diese erhöhte Beweglichkeit der beiden Knochen führt durch Inkongruenz der Gelenkflächen zu einer raschen Arthrose im Handgelenk.

Die Behandlung einer SL-Band-Verletzung kann sowohl konservativ wie auch operativ erfolgen. Bei konservativer Behandlung wird eine sechswöchige Ruhigstellung in einem Gips verordnet. Bei operativer Behandlung wird in der Regel das Band offen genäht. Um ein Auseinanderweichen der Knochen zu verhindern, werden sie für die Dauer der Heilung verschraubt oder durch Drähte miteinander verbunden. Die Schrauben oder Drähte werden anschließend wieder entfernt. Eine Verschraubung der beiden Knochen führt jedoch zu versteifenden Knochenbrücken und engt die physiologisch wichtigen Translationsbewegungen der beiden Knochen bei allen Handgelenksbewegungen auf Dauer ein. Dies führt wiederum zur Arthrose des Handgelenks.

### TECHNISCHE AUFGABE

Ausgehend davon ist es die Aufgabe der vorliegenden Erfindung, ein Implantat bereitzustellen, das zuverlässig zur Behandlung einer SL-Band-Verletzung einsetzbar ist und Schädigungen des Handgelenks, insbesondere Arthrose im Handgelenk, zu vermeiden hilft.

### TECHNISCHE LÖSUNG

Diese Aufgabe wird gelöst durch die Bereitstellung eines Implantats nach Anspruch 1. Vorteilhafte Ausgestaltungen der Erfindung ergeben sich aus den Merkmalen der abhängigen Ansprüche.

Ein erfindungsgemäßes Implantat zur Herstellung einer Bandverbindung zwischen einem ersten Knochen und einem zweiten Knochen umfasst: eine erste Implantatkomponente zur Befestigung am ersten Knochen; eine zweite Implantatkomponente zur Befestigung am zweiten Knochen; und ein Verbindungselement mit einem ersten Anschlussabschnitt und einem zweiten Anschlussabschnitt; wobei die erste Implantatkomponente einen ersten Verbindungsabschnitt zur Herstellung einer Verbindung mit dem ersten Anschlussabschnitt des Verbindungselements, und die zweite Implantatkomponente einen zweiten Verbindungsabschnitt zur Herstellung einer Verbindung mit dem zweiten Anschlussabschnitt des Verbindungselements aufweist. Die Verbindung zwischen dem ersten Verbindungsabschnitt und dem ersten Anschlussabschnitt als um eine erste Drehachse drehbare Verbindung, und/oder die Verbindung zwischen dem zweiten Verbindungsabschnitt und dem zweiten Anschlussabschnitt als um eine zweite Drehachse drehbare Verbindung ausgebildet.

Das Implantat (auch als "Bandschraube" bezeichnet) besteht im Wesentlichen aus zwei kanülierten (d. h. mit jeweils einer durchgängigen Bohrung versehenen) Implantatkomponenten mit Spongiosagewinde von je etwa 5 mm Länge und einem Verbindungselement aus Faden- oder Schlauchmaterial von etwa 10 mm Länge, das beidseitig mit jeweils einer der Implantatkomponenten verbunden ist. Die Implantatkomponenten mit Schraubengewinde sind gegenüber der Faden- bzw. Schlauchverankerung drehbar gelagert. Durch ein Ein- bzw. Ausdrehen aus dem Knochen kann eine gezielte Verschmälerung bzw. Verbreiterung des mit dem Verbindungselement überbrückten Knochenspaltes erreicht werden.

Insbesondere kann das Implantat bei frischen und alten Rupturen des SL-Bandes (scapholunären Bandes; Bandverbindung zwischen Os scaphoideum und Os lunatum) Verwendung finden. Das Os scaphoideum und das Os lunatum sind durch ein schmales Band verbunden, das die Bewegungen der beiden Knochen bei allen Bewegungen des Handgelenkes in bestimmten Grenzen führt und hierdurch an die Gelenkflächen anpasst.

Mit Hilfe des Implantats wird es ermöglicht, eine Ruptur des Bandes zu heilen und so weitere Schädigungen wie Arthrose zu verhindern. Das Implantat kann minimal invasiv (z. B. arthroskopisch assistiert) eingebracht werden und das rupturierte SL-Band für die Zeit der Heilung ersetzen. Die Schraubenköpfe können jeweils in den benachbarten Knochen liegen. Das die Schraubengewinde verbindende Verbindungselement aus Band- oder Fadenmaterial liegt in unmittelbarer Nähe des rupturierten Bandes. Die Knochen werden durch die Bandschraube so weit aneinander angenähert, dass sich die rupturierten Bandränder innig berühren. Jedoch werden die beiden Knochen nicht so nahe zueinander geführt, dass sie in ihrer Bewegung blockiert sind. So kann das Band in annähernd ursprünglichem Abstand vernarben und ausheilen.

Nach der Heilungsphase kann das Handgelenk wieder mit zunehmender Belastung bewegt werden und den ursprünglichen Bewegungsumfang erreichen, ohne dass die Bandschraube entfernt werden muss, da zwischen den beiden Knochen nur der bewegliche Bandanteil verbleibt, der die Stabilität unterstützt und die Bewegungen der verbundenen Knochen nicht blockiert. Selbstverständlich kann die Bandschraube auch entfernt werden, sobald der Heilungsprozess abgeschlossen ist.

Vorzugsweise umfasst das Verbindungselement flexibles Material bzw. besteht aus diesem. Im Bereich der Anschlussabschnitte kann das Material in gewissem Maß formstabil sein, sodass eine haltbare, wenn auch drehbare Verbindung mit der ersten bzw. zweiten Implantatkomponente hergestellt werden kann. So kann das Verbindungselement mit ähnlichen Eigenschaften konzipiert sein wie das SL-Band, das es während der Heilungsphase ersetzt bzw. unterstützt. Die Anschlussabschnitte der Verbindungselemente können in beliebiger Weise ausgebildet sein, um die drehbare Verbindung zur ersten bzw. zweiten Implantatkomponente herzustellen. So kann der Anschlussabschnitt als Schnappverbindung, als geschlossener Ring, der formschlüssig mit der Implantatkomponente in Eingriff gebracht wird, o. ä., ausgebildet sein. Das Verbindungselement ist auf jeden Fall in seinem mittleren Bereich, der das Band ersetzt bzw. unterstützt, flexibel ausgebildet. An den Anschlussabschnitten kann das Verbindungselement ebenfalls flexibel, aber auch weniger flexibel, sogar relativ starr und formstabil, ausgebildet sein.

Insbesondere umfasst das Verbindungselement Fadenmaterial und/oder flexibles Kunststoffmaterial (Schlauchmaterial). Das Verbindungselement kann aus resorbierbarem Material (z. B. Fadenmaterial) hergestellt sein.

Insbesondere weist die erste Implantatkomponente ein erstes Schraubengewinde zum Verschrauben im ersten Knochen und/oder die zweite Implantatkomponente ein zweites Schraubengewinde zum Verschrauben im zweiten Knochen auf. Die Schrauben sind als Spongiosaschrauben, d. h. die Gewinde sind als Spongiosagewinde ausgebildet, die sich für den genannten Anwendungsfall der SL-Bandverbindung besonders eignen. Für andere Anwendungen kann das System jedoch auch andere Gewinde wie Kortikalisgewinde aufweisen.

Die erste und zweite Implantatkomponente können als Lochschrauben oder kanülierte (d. h. mit einem durchgehenden Kanal versehene) Schrauben ausgebildet sein. Der Zweck des Kanals ergibt sich aus der Operationstechnik. Zunächst wird ein Draht verwendet, um die Position der Implantatkomponenten festzulegen. Ist der Draht entsprechend positioniert, wird ein kanülierter Bohrer über den Draht geführt, um die Löcher für den Einsatz der Implantatkomponenten zu bohren. Anschließend werden letztere über den Draht geführt und mittels eines (ebenfalls kanülierten, über den Draht geführten) Schraubwerkzeugs in den jeweiligen Knochen verschraubt zu werden. Anschließend wird das Werkzeug so weit zurückgezogen, dass es lediglich an eine der beiden Implantatkomponenten angreift. Diese kann nun in die eine oder andere Richtung gedreht werden, um den Abstand zur anderen Implantatkomponente einzustellen bzw. zu justieren. Nachdem der gewünschte Abstand hergestellt ist, werden das Werkzeug und der Draht entfernt.

Sie können insbesondere Titan umfassen bzw. aus Titan oder einer Titanlegierung hergestellt sein. Es ist jedoch auch denkbar, andere Materialien, z. B. bioresorbierbare Materialien (z. B. Biopolymere) zu verwenden. Die Anforderungen von Bio-Kompatibilität und Festigkeit der Schrauben müssen jedenfalls gewährleistet sein.

Vorzugsweise kann das erste Schraubengewinde durch Drehen der ersten Implantatkomponente um eine erste Schraubenachse und/oder das zweite Schraubengewinde durch Drehen der zweiten Implantatkomponente um eine zweite Schraubenachse mit dem ersten Knochen bzw. mit dem zweiten Knochen verschraubbar sein. Die Schraubenachsen bilden die zentralen Achsen der Gewinde. Die Implantatkomponenten können mit einem selbst schneidenden Gewinde, evtl. einem rechtsdrehenden Gewinde, versehen sein.

Es ist besonders bevorzugt, dass durch die Erfindung die Breite des durch das Implantat überbrückten Spalts zwischen dem ersten Knochen und dem zweiten Knochen durch Hinein- bzw. Herausdrehen der ersten Implantatkomponente um die erste Schraubenachse und/oder durch Hinein- bzw. Herausdrehen der zweiten Implantatkomponente um die zweite Schraubenachse veränderbar ist. Eine wichtige Eigenschaft der Erfindung besteht darin, dass die erste und/oder zweite Implantatkomponente in gewissen Grenzen mehr oder weniger weit in die Spongiosa eingedreht werden können, um so den Abstand zwischen den zu verbindenden Knochen an die Erfordernisse für die Bandheilung anzupassen.

Vorzugsweise liegen die erste Drehachse und die erste Schraubenachse auf einer ersten Geraden liegen, und/oder die zweite Drehachse und die zweite Schraubenachse auf einer zweiten Geraden. D. h. die entsprechenden Achsen fluchten bzw. fallen zusammen. Auf diese Weise kann eine erste oder zweite Implantatkomponente in den Knochen hinein (oder heraus) gedreht werden, während sich das Verbindungselement relativ zum umgebenden Gewebe und Knochen nicht dreht, also in seiner Lage verharrt. Somit ist eine Feinjustierung möglich, ohne das Verbindungselement von den Schrauben zu lösen.

Die erste Implantatkomponente kann insbesondere einen ersten Ansatz zum Eingriff eines Schraubwerkzeugs, und/oder die zweite Implantatkomponente einen zweiten Ansatz zum Eingriff eines Schraubwerkzeugs aufweisen. Die Eingriffe bzw. Anschlüsse können beliebig ausgebildet sein, z. B. als Sechskant, Kreuzschlitz, um das Eindrehen der ersten und zweiten Implantatkomponenten in den Knochen zu ermöglichen. Es ist auch gleichgültig, ob ein männlicher oder weiblicher Anschluss bzw. Eingriff vorgesehen ist. Vorzugsweise ist jedoch der Anschluss oder Eingriff an den Verbindungsabschnitten ausgebildet, z. B. als Innengewinde in einer Bohrung des jeweiligen Verbindungsabschnitts.

Der erste Verbindungsabschnitt kann eine erste ringförmige Vertiefung zum Eingriff des ersten Anschlussabschnitts und/oder der zweite Verbindungsabschnitt kann eine zweite ringförmige Vertiefung zum Eingriff des zweiten Anschlussabschnitts aufweisen. Die Vertiefung ist ringförmig, d. h. die Außenkontur des Verbindungsabschnitts weist zumindest abschnittsweise entlang der Schraubenachse einen kreisförmigen Querschnitt auf. Dieser kann konstant oder variabel sein. Die Vertiefung wird in axialer Richtung durch einen Vorsprung, der als Anschlag für den ersten oder zweiten Anschlussabschnitt dient, begrenzt.

Der erste Anschlussabschnitt weist insbesondere ein erstes ringförmiges Eingriffelement und/oder der zweite Anschlussabschnitt eine zweites ringförmiges Eingriffelement auf. Die Eingriffelemente entsprechen so den Vertiefungen der Verbindungsabschnitte, dass sie mit diesen in gegenseitigen Eingriff gebracht bzw. mit diesen verrastet werden können. Die Verbindung ist durch die Ausgestaltung drehbar, da sich die ringförmigen Eingriffelemente auf den ringförmigen Vertiefungen in radialer Richtung bewegen (also drehen) können.

Ein ringförmiges Eingriffelement muss sich nicht über den gesamten Kreisumfang erstrecken bzw. muss nicht über den gesamten Kreisumfang geschlossen sein, sondern kann an einer oder and mehreren Stellen unterbrochen sein. Das Eingriffelement verläuft somit entlang eines oder mehrerer Kreisabschnitte, z. B. in Form eines Zylinderabschnitts, der sich in axialer Richtung erstreckende Öffnungen aufweisen kann

Vorzugsweise weist das erste ringförmige Eingriffelement eine erste Ausnehmung und/oder das zweite ringförmige Eingriffelement eine zweite Ausnehmung auf. Dies ist sinnvoll, um eine kurzfristige elastische Aufweitung der Eingriffelemente zu ermöglichen, um dieses beim Befestigungsvorgang über den als Anschlag dienenden Vorsprung der ersten bzw. zweiten Implantatkomponente zu führen und anschließend in der Vertiefung einzurasten. Die Ausnehmung erstreckt sich vornehmlich axial bzgl. der ringförmigen Eingriffelemente, d. h. die Ausnehmung reicht über eine gesamte axiale Länge des Eingriffelements. Das Einrasten kann durch Formstabilität und Elastizität der Anschlussabschnitte erfolgen.

Es kann jedoch, wenn das Verbindungselement aus deformierbarem, aber nicht elastischem Material besteht, auch vorgesehen sein, dass die Komponenten nach dem Zusammenfügen bzw. der relativen Positionierung der Komponenten zueinander durch ein Schließen bzw. Überbrücken der Ausnehmung aneinander befestigt werden.

Das Verbindungselement kann vorzugsweise wenigstens einen Überbrückungsabschnitt zur Überbrückung des zwischen dem ersten Knochen und dem zweiten Knochen zu überbrückenden Spalts aufweisen, wobei sich der Überbrückungsabschnitt im Wesentlichen linear zwischen dem ersten Anschlussabschnitt und dem zweiten Anschlussabschnitt erstreckt Wenigstens die Überbrückungsabschnitte des Verbindungselements sind flexibel ausgebildet. Es sind insbesondere zwei, vier oder mehrere lineare Überbrückungsabschnitte vorgesehen, die das SL-Band ersetzen bzw. unterstützen. Die Anzahl und Lage hängt von den physiologischen Erfordernissen ab. Beispielsweise kann das Verbindungselement aus mehreren bandartig geflochtenen Fäden (z. B. 10 mm Länge und 3 mm Breite) bestehen.

Für die beschriebenen Merkmale soll sowohl einzeln als auch in beliebiger Kombination mit anderen Merkmalen Schutz beansprucht werden, unabhängig davon, ob einzelne Merkmale nur in Kombination mit anderen Merkmalen oder nicht in Kombination mit bestimmten anderen Merkmalen beschrieben wurden.

### KURZE BESCHREIBUNG DER ZEICHNUNGEN

Weitere Merkmale, Eigenschaften und Vorteile der vorliegenden Erfindung werden anhand der Beschreibung spezieller Ausführungsbeispiele anhand der nachfolgenden Zeichnungen verdeutlich. Es zeigen:
Figur 1: eine perspektivische Ansicht der Komponenten eines erfindungsgemäßen Implantats; und
Figur 2: eine perspektivische Ansicht des erfindungsgemäßen Implantats.

### DETAILLIERTE BESCHREIBUNG EINER AUSFÜHRUNGSFORM DER ERFINDUNG

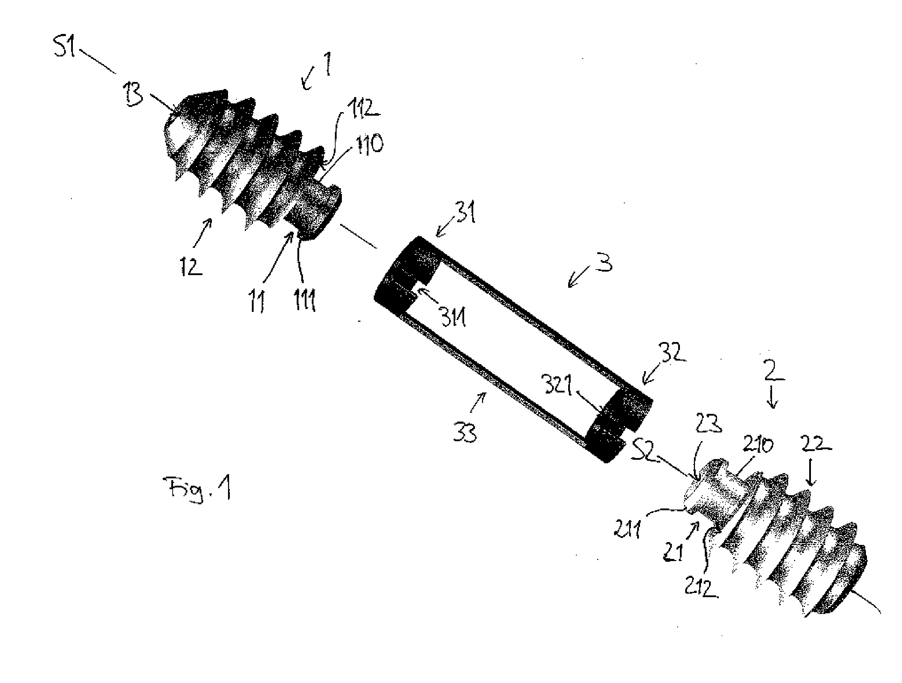

Die Figur 1 zeigt die Komponenten 1, 2 und 3 eines erfindungsgemäßen Implantats (Bandschraube) in getrenntem Zustand.

Die erste Implantatkomponente 1 und die zweite Implantatkomponente 2 weisen gleichen Aufbau auf und werden daher zusammen beschrieben.

Die erste bzw. zweite Implantatkomponente 1 bzw. 2 weisen jeweils einen Verbindungsabschnitt 11 bzw. 21 zur Verbindung mit der dritten Implantatkomponente 3 sowie ein Schraubengewinde 12 bzw. 22, insbesondere ein Spongiosa-Schraubengewinde, auf. Die Implantatkomponenten 1 und 2 sind als Lochschrauben mit einer durchgehenden Öffnung 13 bzw. 23 ausgeführt.

Die Verbindungsabschnitte 11 und 21 weisen jeweils einen ring- bzw. zylindermantelförmigen Eingriffabschnitt 110 bzw. 210 und einen ringförmigen Vorsprung 111 bzw. 211 auf. Die Vorsprünge 111 bzw. 211 grenzen jeweils den vertieften Eingriffabschnitt 110 bzw. 210 in eine Richtung entgegengesetzt zum Schraubengewinde 12 bzw. 22 hin ab. Die schraubengewindeseitige Abgrenzung bildet ein Anschlag 112 bzw. 212. Die Vorsprünge 111, 211 grenzen eine Nut ab und bilden eine schienenartigen Verankerung, die eine Drehbewegung erlaubt.

Das Schraubengewinde 12 bzw. 22 wird mehr oder weniger weit in die Spongiosa des Os scaphoideum bzw. des Os lunatum eingeschraubt. Dabei wird die Schraube um die Schraubenachse S 1 bzw. S2 gedreht. Durch die Tiefe des Einschraubens der ersten bzw. zweiten Implantatkomponente 1 bzw. 2 wird die Lage des Verbindungsabschnitts 11 bzw. 21 und damit der Abstand der beiden Verbindungsabschnitte 11 und 21 voneinander eingestellt.

Das Verbindungselement 3 besteht im Wesentlichen aus einem ersten Anschlussabschnitt 31, einem zweiten Anschlussabschnitt 32, und einem Überbrückungsabschnitt 33. Es ist aus flexiblem, biokompatiblen Material hergestellt.

Der erste und zweite Anschlussabschnitt 31 und 32 sind jeweils als Halteringe mit einem axialen Durchbruch 311 bzw. 321 ausgebildet. Statt Halteringen können auch schlaufenartige Anschlussabschnitte vorgesehen sein, die für eine drehbare, formschlüssige Verbindung sorgen. Der Innendurchmesser der Halteringe entspricht in etwa dem Außendurchmesser der Eingriffabschnitte 110 bzw. 210.

Die Halteringe sind durch zwei lineare Abschnitte 331 und 332 des Überbrückungsabschnitts 33 miteinander verbunden. Wenigstens der Überbrückungsabschnitt 33 ist flexibel derart ausgebildet, dass er ein rupturiertes SL-Band für die Zeit der Heilung ersetzen kann, und dabei eine physiologisch natürliche Beweglichkeit des Os scaphoideum gegenüber dem Os lunatum zulässt.

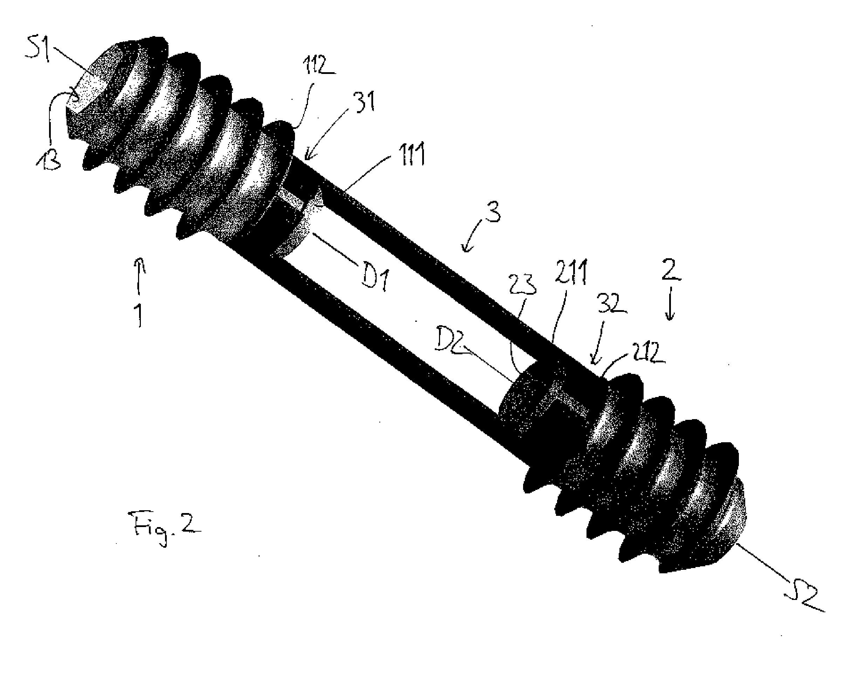

In der Figur 2 ist das Implantat im zusammengesetzten Zustand dargestellt. Der ringförmige erste und zweite Anschlussabschnitt 31 und 32 ist jeweils in dem vertieften Eingriffabschnitt 110 bzw. 210 angeordnet. Die Fixierung in axialer Richtung erfolgt durch ein Anliegen der Halteringe 31 bzw. 32 an den Vorsprüngen 111 bzw. 211, die als Anschlag gegenüber Zugbelastung dienen, und den Anschlägen 112 bzw. 212, die gegen Druckbelastung wirken. Die Halteringe 31 und 32 (oder andere Eingriffelemente) sind somit jeweils drehbar, formschlüssig in den Eingriffabschnitten 110 bzw. 210 aufgenommen.

Die Durchbrüche 311 bzw. 321 (vgl. Figur 1) erlauben eine kurzzeitige elastische Aufweitung der Halteringe 31 bzw. 32, sodass diese beim Verbindungsvorgang mit der ersten bzw. zweiten Implantatkomponente über die Vorsprünge 111 bzw. 211 geführt werden können. Nach der elastischen Entspannung nehmen die Halteringe 31 und 32 den in der Figur 2 dargestellten axial fixierten Zustand ein. Die Halteringe 31 und 32 werden auf die Verbindungsabschnitte 11 bzw. 21 aufgeclipst bzw. verrasten in diesen.

Durch die Art der Verbindung wird gewährleistet, dass die erste Implantatkomponente 1 gegenüber dem ersten Anschlussabschnitt 31 um eine erste Drehachse D1 drehbar an diesem befestigt ist. In ähnlicher Weise ist die zweite Implantatkomponente 2 gegenüber dem zweiten Anschlussabschnitt 32 um eine erste Drehachse D2 drehbar an diesem befestigt. Insbesondere fällt die Schraubenachse S 1 mit der Drehachse D 1 und die Schraubenachse S2 mit der Drehachse D2 zusammen, d. h. sie liegen jeweils auf einer Geraden. Die Implantatkomponenten 1 und 2 sind also axial fixiert, aber jeweils drehbar mit dem jeweiligen Anschlussabschnitt 31 bzw. 32 des Verbindungselements 3 verbunden. Dies ermöglicht ein Einschrauben der Implantatkomponenten 1 und 2 in den Knochen, wobei das Verbindungselement 3 bereits an der jeweiligen ersten und/oder zweiten Implantatkomponente 1 bzw. 2 befestigt sein kann. Durch Einschrauben bzw. Herausschrauben ist ein Ein- und Nachstellen des Abstands der Verbindungsabschnitte 11 und 21 möglich. Der Abstand kann so an den zu überbrückenden Zwischenraum zwischen den zu verbindenden Knochen angepasst werden.

In den durchgehenden Öffnungen 13 und 23 ist ein (durchgehendes) 6-Kant-Gewinde zum Eingriff eines Imbusschlüssels o. ä. ausgebildet. Dieses dient zum Eingriff des Werkzeugs, um die Implantatkomponenten 1 und 2 Ein- bzw. Auszuschrauben und so den Abstand der Verbindungsabschnitte 11 und 21 zu justieren.

## Patentansprüche

1. Implantat zur Herstellung einer Bandverbindung zwischen einem ersten Knochen und einem zweiten Knochen, umfassend:
eine erste Implantatkomponente (1) zur Befestigung am ersten Knochen;
eine zweite Implantatkomponente (2) zur Befestigung am zweiten Knochen; und
ein Verbindungselement (3) mit einem ersten Anschlussabschnitt (31) und einem zweiten Anschlussabschnitt (32);
wobei die erste Implantatkomponente (1) einen ersten Verbindungsabschnitt (11) zur Herstellung einer Verbindung mit dem ersten Anschlussabschnitt (31) des Verbindungselements (3), und die zweite Implantatkomponente (2) einen zweiten Verbindungsabschnitt (21) zur Herstellung einer Verbindung mit dem zweiten Anschlussabschnitt (32) des Verbindungselements (3) aufweist;
**dadurch gekennzeichnet, dass**
die Verbindung zwischen dem ersten Verbindungsabschnitt (11) und dem ersten Anschlussabschnitt (31) als um eine erste Drehachse (D1) drehbare Verbindung, und/oder die Verbindung zwischen dem zweiten Verbindungsabschnitt (21) und dem zweiten Anschlussabschnitt (32) als um eine zweite Drehachse (D2) drehbare Verbindung ausgebildet ist.

2. Implantat nach Anspruch 1,
**dadurch gekennzeichnet, dass**
das Verbindungselement (3) flexibles Material umfasst.

3. Implantat nach Anspruch 1 oder 2,
**dadurch gekennzeichnet, dass**
das Verbindungselement (3) Fadenmaterial und/oder flexibles Kunststoffmaterial umfasst.

4. Implantat nach einem der vorhergehenden Ansprüche,
**dadurch gekennzeichnet, dass**
die erste Implantatkomponente (1) ein erstes Schraubengewinde (11) zum Verschrauben im ersten Knochen und/oder die zweite Implantatkomponente (2) ein zweites Schraubengewinde (21) zum Verschrauben im zweiten Knochen umfasst.

5. Implantat nach Anspruch 4,
**dadurch gekennzeichnet, dass**
das erste Schraubengewinde (12) durch Drehen der ersten Implantatkomponente (1) um eine erste Schraubenachse (S 1) und/oder das zweite Schraubengewinde (22) durch Drehen der zweiten Implantatkomponente (2) um eine zweite Schraubenachse (S2) mit dem ersten Knochen bzw. mit dem zweiten Knochen verschraubbar ist.

6. Implantat nach Anspruch 5,
**dadurch gekennzeichnet, dass**
die Breite des durch das Implantat überbrückten Spalts zwischen dem ersten Knochen und dem zweiten Knochen durch Hinein- bzw. Herausdrehen der ersten Implantatkomponente (1) um die erste Schraubenachse (S1) und/oder durch Hinein- bzw. Herausdrehen der zweiten Implantatkomponente (2) um die zweite Schraubenachse (S2) veränderbar ist.

7. Implantat nach Anspruch 6,
**dadurch gekennzeichnet, dass**
die erste Drehachse (D1) und die erste Schraubenachse (S1) auf einer ersten Geraden liegen, und/oder die zweite Drehachse (D2) und die zweite Schraubenachse (S2) auf einer zweiten Geraden liegen.

8. Implantat nach einem der vorhergehenden Ansprüche,
**dadurch gekennzeichnet, dass**
die erste Implantatkomponente (1) einen ersten Ansatz zum Eingriff eines Schraubwerkzeugs, und/oder die zweite Implantatkomponente einen zweiten Ansatz zum Eingriff eines Schraubwerkzeugs aufweist.

9. Implantat nach einem der vorhergehenden Ansprüche,
**dadurch gekennzeichnet, dass**
der erste Verbindungsabschnitt (11) eine erste ringförmige Vertiefung zum Eingriff des ersten Anschlussabschnitts (31) und/oder der zweite Verbindungsabschnitt (21) eine zweite ringförmige Vertiefung zum Eingriff des zweiten Anschlussabschnitts (32) aufweist.

10. Implantat nach einem der vorhergehenden Ansprüche,
**dadurch gekennzeichnet, dass**
der erste Anschlussabschnitt (31) ein erstes ringförmiges Eingriffelement (310) und/oder der zweite Anschlussabschnitt (32) eine zweites ringförmiges Eingriffelement (320) aufweist.

11. Implantat nach Anspruch 10,
**dadurch gekennzeichnet, dass**
das erste ringförmige Eingriffelement (310) eine erste Ausnehmung (311) und/oder das zweite ringförmige Eingriffelement (320) eine zweite Ausnehmung (321) aufweist.

12. Implantat nach einem dr vorhergehenden Ansprüche,
**dadurch gekennzeichnet, dass**
das Verbindungselement (3) wenigstens einen Überbrückungsabschnitt (33) zur Überbrückung des zwischen dem ersten Knochen und dem zweiten Knochen zu überbrückenden Spalts aufweist, wobei sich der Überbrückungsabschnitt im Wesentlichen linear zwischen dem ersten Anschlussabschnitt (31) und dem zweiten Anschlussabschnitt (32) erstreckt.
